# EUROPÄISCHE PATENTSCHRIFT

(11) **EP 1 289 502 B1**
(45) Veröffentlichungstag und Bekanntmachung des Hinweises auf die Patenterteilung: **19.07.2006**
(21) Anmeldenummer: 00967491.2
(22) Anmeldetag: 20.10.2000
(51) Int. Cl.: A61K 9/107, A61K 9/48, A61K 38/13

(54) **MIKROEMULSION-PREKONZENTRAT, MIKROEMULSION UND VERWENDUNG EINER ZUSAMMENSETZUNG**
MICROEMULSION PRECONCENTRATES, MICROEMULSION AND USE OF SUCH A COMPOSITION
PRECONCENTRE A MICROEMULSIONS, MICROEMULSION ET UTILISATION D'UNE COMPOSITION DE CE TYPE

(30) Priorität: 20.10.1999 CH 191299
(43) Veröffentlichungstag der Anmeldung: 12.03.2003
(73) Patentinhaber: VESIFACT AG, 6342 Baar 2 (CH)
(72) Erfinder: SUPERSAXO, Andreas, Werner, CH-6340 Baar (CH); WEDER, Marc, Antoine, CH-8803 Rüschlikon (CH); WEDER, Hans, Georg, CH-8803 Rüschlikon (CH)
(74) Vertreter: Welch, Andreas
(86) Internationale Anmeldenummer: PCT/CH2000/000569
(87) Internationale Veröffentlichungsnummer: WO 2001/028520

(56) Entgegenhaltungen:
- WO-A-93/18852
- WO-A-99/29300
- WO-A-99/49848
- WO-A-99/56727

## Beschreibung

Die vorliegende Erfindung bezieht sich auf neue Zusammensetzungen und die Verwendung einer Zusammensetzung gemäss Oberbegriff der unabhängigen Ansprüche.

In einer bevorzugten Ausführungsform der vorliegenden Erfindung wird ein in Wasser schwer löslicher Wirkstoff verwendet. Cyclosporine sind ein Beispiel für solche schwer löslichen Wirkstoffe.

Die Cyclosporine umfassen eine Klasse cyclischer poly-N-methylierter Undecapeptide, deren Vertreter über pharmakologische Eigenschaften verfügen, insbesondere über immunosuppressive, antiinflammatorische und/oder antiparasitische Eigenschaften. Der bekannteste Vertreter dieser Klasse ist das Cyclosporin A, welches der Wirkstoff der Handelspräparate SANDIMMUN und NEORAL ist.

Die Cyclosporine sind neutrale lipophile (d.h. hydrophobe) Substanzen mit geringer Wasserlöslichkeit. Formulierungen von Cyclosporinen, z.B. für eine orale Verabreichung, beruhten vorwiegend auf der Anwendung von Ethanol und Ölen oder ähnlichen Hilfsstoffen als Trägermedien. So ist gemäss Journal of Drug Targeting, 1998, Vol. 5, No. 6, Seiten 443-458 (insbesondere Seiten 447 und 448) das Handelspräparat SANDIMMUN in drei verschiedenen Formen erhältlich, nämlich
- Weichgelatinekapseln, enthaltend Cyclosporin A, wasserfreien Ethylalkohol (bis zu 12.7%), Labrafil M 2125 CS (polyoxyethylierte glycolisierte Glyceride), Maisöl und weitere Ingredientien;
- eine orale Lösung, enthaltend Cyclosporin A, Ethylalkohol (bis zu 12.5%), Olivenöl, Labrafil M 1944 CS (polyoxyethylierte Ölsäureglyceride) und weitere Ingredientien; und
- ein Injektionspräparat, enthaltend Cyclosporin A, Ethylalkohol (32.9%) und Cremophor EL (polyethoxyliertes Rizinusöl); und das Handelspräparat NEORAL in zwei Formen, nämlich
- Weichgelatinekapseln, enthaltend Cyclosporin A, wasserfreien Ethylalkohol (bis zu 9.5%), Maisölglyceride, Cremophor RH 40 (polyoxyethyliertes hydriertes Rizinusöl), Propylenglykol und weitere Ingredientien; und
- eine orale Lösung, enthaltend Cyclosporin A, wasserfreien Ethylalkohol (bis zu 9.5%), Maisölglyceride, Cremophor RH 40, Propylenglykol und weitere Excipientien.

Aus DE 39 30 928 C2 sind Formulierungen bekannt, welche ein Cyclosporin als Wirkstoff; eine hydrophile Phase, nämlich einen pharmazeutisch annehmbaren C₁-C₅-Alkyl- oder Tetrahydrofurfuryl-Diether oder -Teilether eines niedermolekularen Mono- oder Polyoxy-C₂-C₁₂-alkandiols, vorzugsweise Transcutol (Diethylenglykolmonoethylether) bzw. Glycofurol (Tetrahydrofurfurylalkoholpolyethylenglykolether), und/oder 1,2-Propylenglykol; eine lipophile Phase; und ein oberflächenaktives Mittel enthalten. Die hydrophile Phase kann zusätzlich auch noch weitere Komponenten enthalten, beispielsweise niedere Alkanole, wie Ethanol.

Aus WO 99/00002 A sind Formulierungen bekannt, welche ein Cyclosporin als Wirkstoff; einen Polycarbonsäureester und/oder einen Polyolcarbonsäureester; ein Öl; und ein oberflächenaktives Mittel enthalten. Bei den Polycarbonsäureestern handelt es sich dabei um Veresterungsprodukte von Polycarbonsäuren mit 2-10, vorzugsweise 3-5 Carboxylgruppen mit C₁-C₁₀-Alkoholen, wie Triethylcitrat, Tributylcitrat, Acetyltributylcitrat und Acetyltriethylcitrat, und bei den Polyolcarbonsäureestern um Veresterungsprodukte von Polyolen mit 2-10, vorzugsweise 3-5 Hydroxylgruppen mit C₂-C₁₁-Carbonsäuren, wie z.B. Triacetin.

Wie bei den vorstehend diskutierten bekannten Formulierungen handelt es sich bei den Formulierungen gemäss vorliegender Erfindung um Mikroemulsion-Prekonzentrate bzw. Mikroemulsionen.

Unter einem Mikroemulsion-Prekonzentrat wird ein System verstanden, das beim Kontakt mit Wasser, z.B. bei Zugabe zu Wasser, eine Mikroemulsion ergibt. Bei einer solchen Mikroemulsion handelt es sich im herkömmlich anerkannten Sinn um eine nicht-opaque oder praktisch nicht-opaque kolloidale Dispersion, welche Wasser und organische Komponenten unter Einschluss lipophiler (d.h. hydrophober) Komponenten enthält.

Mikroemulsionen lassen sich dadurch erkennen, dass sie eine oder mehrere der folgenden Eigenschaften aufweisen:
- Sie werden spontan gebildet, wenn ihre Komponenten miteinander in Kontakt gebracht werden; es ist also hierzu praktisch keine Zufuhr von Energie notwendig, und die Bildung solcher Mikroemulsionen erfolgt daher ohne Erhitzen oder Anwendung einer hohen Scherkraft oder einer anderen wesentlichen Durchmischung.
- Sie sind praktisch nicht opaque, nämlich transparent oder opaleszent, wenn sie unter einem optischen Mikroskop betrachtet werden. Sie sind in ihrem nichtgestörten Zustand optisch isotrop, obwohl sich bei einer Beobachtung beispielsweise unter Anwendung einer Röntgenstrahltechnik eine anisotrope Struktur feststellen lässt.
- Sie enthalten eine disperse oder stückige (Tröpfchen-) Phase, deren Teilchen eine Grösse von weniger als 200 nm haben, wovon ihre optische Transparenz herrührt. Die Teilchen können kugelig sein oder auch sonstige Strukturen haben; beispielsweise können sie flüssige Kristalle mit lamellaren, hexagonalen oder isotropen Symmetrien sein. Im allgemeinen enthalten Mikroemulsionen Tröpfchen oder Teilchen mit einer maximalen Abmessung, beispielsweise einem Durchmesser, von weniger als 150 nm, gewöhnlich etwa 10-100 nm.

Bei den erfindungsgemässen Mikroemulsion-Prekonzentraten handelt es sich demgemäss vor allem um Systeme, die einen in Wasser schwerlöslichen Wirkstoff, wie ein Cyclosporin enthalten und beim Zusammenbringen mit Wasser spontan oder praktisch spontan, d.h. ohne nennenswerten Energieeintrag, zur Bildung einer Mikroemulsion befähigt sind.

Die erfindungsgemässen Mikroemulsion-Prekonzentrate sind vor allem dadurch gekennzeichnet, dass sie
(a) eine Mischung bestehend aus einem mittelkettigen Triglycerid und einer Omega-9-Fettsäure und/oder einer Omega-6-Fettsäure und
(b) eine oberflächenaktive Komponente enthaltend ein Tensid vom Polyoxethylen-Typ. Solche Prekonzentrate enthalten vorzugsweise als Komponente (c) einen in Wasser schwerlöslichen, in Komponente (a) und/oder (b) jedoch löslichen Wirkstoff.

Im Gegensatz zu den Formulierungen gemäss DE 39 30 928 C2 und WO 99/00002 A können sie im wesentlichen frei von mit Wasser mischbaren oder in Wasser löslichen Komponenten sein. Dabei handelt es sich insbesondere um die Komponenten
(a) C₁-C₅-Alkyl- oder Tetrahydrofurfuryl-Diether oder -Teilether niedermolekularer Mono- oder Polyoxy-C₂-C₁₂-Alkandiole;
(b) 1,2-Propylenglykol;
(c) niedere Alkanole;
(d) Veresterungsprodukte von Polycarbonsäuren mit 2-10, insbesondere 3-5 Carboxylgruppen mit C₁-C₁₀-Alkoholen; und
(e) veresterungsprodukte von Polyolen mit 2-10, insbesondere 3-5 Carboxylgruppen mit C₂-C₁₁-Carbonsäuren;
insbesondere im wesentlichen frei von Diethylenglykolmonomethylether, Glycofurol, 1,2-Propylenglykol, Triethylcitrat, Tributycitrat, Acetyltributycitrat, Acetyltriethylcitrat, Triacetin, Ethanol, Polyethylenglykol, Dimethylisosorbit und Propylencarbonat.

Als Komponente (a) eignen sich Mischungen aus einem mittelkettigen Fettsäuretriglycerid, zweckmässigerweise einem Fettsäuretriglycerid, in welchem die Fettsäurereste 4 bis 18, vorzugsweise 6 bis 18 C-Atome aufweisen, und einer Omega-9- und/oder einer Omega-6-Fettsäure. Diese Substanzen sind mit Wasser nicht mischbar oder in Wasser unlöslich bzw. praktisch unlöslich und weisen keine oder praktisch keine oberflächenaktive Funktion auf.

Bevorzugte mittelkettige Fettsäuretriglyceride sind Capryl-/Caprinsäure-Triglyceride, wie sie beispielsweise unter der Warenbezeichnung MIGLYOL bekannt und im Handel erhältlich sind (Fiedler, Lexikon der Hilfsstoffe, 3. Auflage, Seiten 808 bis 809, 1989). Hierzu gehören beispielsweise die folgenden Produkte:

### MIGLYOL 810

Hierbei handelt es sich um ein fraktioniertes Kokosnussöl, das Triglyceride von Caprylsäure und Caprinsäure enthält und ein Molekulargewicht von etwa 520 hat. Es weist eine Fettsäurezusammensetzung mit C₆ maximal 2 Prozent, C₈ etwa 65 bis 75 Prozent, C₁₀ etwa 25 bis 35 Prozent und C₁₂ maximal 2 Prozent auf.

### MIGLYOL 812

Hierbei handelt es sich um ein fraktioniertes Kokosnussöl, das Triglyceride von Caprylsäure und Caprinsäure enthält und ein Molekulargewicht von etwa 520 hat. Es weist eine Fettsäurezusammensetzung mit C₆ maximal 3 Prozent, C₈ etwa 50 bis 65 Prozent, C₁₀ etwa 30 bis 45 Prozent und C₁₂ maximal 5 Prozent auf.

### MIGLYOL 818

Hierbei handelt es sich um Triglyceride von Caprylsäure, Caprinsäure und Linolsäure mit einem Molekulargewicht von etwa 510 hat. Es weist eine Fettsäurezusammensetzung mit C₆ maximal 3 Prozent, C₈ etwa 45 bis 60 Prozent, C₁₀ etwa 25 bis 40 Prozent, C₁₂ etwa 2 bis 5 Prozent und C_{18:2} etwa 4 bis 6 Prozent Prozent auf.

Ferner sind auch Triglyceride von Caprylsäure und Caprinsäure geeignet, wie sie unter der Warenbezeichnung MYRITOL bekannt und erhältlich sind (Fiedler, Lexikon der Hilfsstoffe, 3. Auflage, Seite 834, 1989). Hierzu gehört beispielsweise das Produkt MYRITOL 813.

Weitere geeignete Produkte dieser Klasse sind CAPTEX 355, CAPTEX 300, CAPTEX 800, CAPMUL MCT, NEOBEE M5 und MAZOL 1400.

Geeignete Omega-9-Fettsäuren sind hauptsächlich solche mit 12-24, insbesondere 16-24, vorzugsweise 18-22 C-Atomen, beispielsweise Oelsäure und Eicosatriensäure. Besonders bevorzugt ist die Oelsäure.

Geeignete Omega-6-Fettsäuren sind hauptsächlich solche mit 12-24, insbesondere 16-24, vorzugsweise 18-22 C-Atomen, beispielsweise Linolsäure, gamma-Linolensäure, dihommo-gamma-Linolensäure und Arachidonsäure. Besonders bevorzugt ist die Linolsäure.

In einer besonders bevorzugten Ausführungsform verwendet man als Komponente (a) eine Mischung bestehend aus einem Capryl-/Caprinsäure-Triglycerid, Oelsäure und/oder Linolsäure.

Komponente (c), der in Wasser schwerlösliche, in Komponente (a) und/oder (b) jedoch lösliche therapeutische Wirkstoff, ist beispielsweise ein Cyclosporin, vorzugsweise Cyclosporin A oder ein anderes geeignetes Cyclosporin, beispielsweise ein anderes natürliches Cyclosporin, eines der verschiedenen nichtnatürlichen Cyclosporinderivate oder ein synthetisches Cyclosporin.

Bei Komponente (b), der oberflächenaktiven Komponente enthaltend ein Tensid vom Polyoxethylen-Typ, kann es sich um ein hydrophiles oberflächenaktives Mittel oder um ein lipophiles oberflächenaktives Mittel handeln, doch kommen auch Gemische solcher Mittel in Frage.

Beispiele für solche Tenside sind folgende:
- Reaktionsprodukte von natürlichen oder hydrierten Pflanzenölen und Ethylenglykol, nämlich polyoxyethylenglykolierte natürliche oder hydrierte Pflanzenöle, wie polyoxyethylenglykolierte natürliche oder hydrierte Rizinusöle. Besonders geeignet sind die verschiedenen Tenside, die unter der Bezeichnung CREMOPHOR bekannt und erhältlich sind (Fiedler, Lexikon der Hilfsstoffe, 3. Auflage, Seiten 326 bis 327, 1989), vor allem die Produkte mit den Bezeichnungen CREMOPHOR RH 40, CREMOPHOR RH 60 und CREMOPHOR EL. Ferner eignen sich als solche Produkte auch die verschiedenen Tenside, die unter der Bezeichnung NIKKOL bekannt und erhältlich sind, beispielsweise NIKKOL HCO-60.
- Polyoxyethylensorbitanfettsäureester, beispielsweise die Mono- und Trilaurylester, die Mono- und Tripalmitylester, die Mono- und Tristearylester und die Mono- und Trioleylester, wie sie unter der Bezeichnung TWEEN bekannt und erhältlich sind (Fiedler, Lexikon der Hilfsstoffe, 3. Auflage, Seiten 1300 bis 1304, 1989), beispielsweise die Produkte
   TWEEN 20: Polyoxyethylen(20)sorbitanmonolaurat,
   TWEEN 40: Polyoxyethylen(20)sorbitanmonopalmitat,
   TWEEN 60: Polyoxyethylen(20)sorbitanmonostearat,
   TWEEN 80: Polyoxyethylen(20)sorbitanmonooleat,
   TWEEN 65: Polyoxyethylen(20)sorbitantristearat,
   TWEEN 85: Polyoxyethylen(20)sorbitantrioleat,
   TWEEN 21: Polyoxyethylen(4)sorbitanmonolaurat,
   TWEEN 61: Polyoxyethylen(4)sorbitanmonostearat und
   TWEEN 81: Polyoxyethylen(4)sorbitanmonooleat.

Besonders bevorzugt aus dieser Klasse von Verbindungen ist TWEEN 80.
- Polyoxyethylenfettsäureester, beispielsweise die im Handel unter der Bezeichnung MYRJ bekannten und erhältlichen Polyoxyethylenstearinsäureester (Fiedler, Lexikon der Hilfsstoffe, 3. Auflage, Seite 834, 1989), insbesondere das Produkt MYRJ 52, sowie auch die unter der Bezeichnung CETIOL HE bekannten und erhältlichen Polyoxyethylenfettsäureester (Fiedler, Lexikon der Hilfsstoffe, 3. Auflage, Seite 284, 1989).
- Copolymerisate von Polyoxyethylen und Polyoxypropylen, wie sie beispielsweise unter den Bezeichnungen PLURONIC und EM-KALYX bekannt und erhältlich sind (Fiedler, Lexikon der Hilfsstoffe, 3. Auflage, Seiten 956 bis 958, 1989), insbesondere das Produkt PLURONIC F68.
- Blockcopolymerisate von Polyoxyethylen und Polyoxypropylen, wie sie beispielsweise unter der Bezeichnung POLOXAMER bekannt und erhältlich sind (Fiedler, Lexikon der Hilfsstoffe, 3. Auflage, Seite 959, 1989), insbesondere das Produkt POLOXAMER 188.
- Polyethoxylierte Vitamin E Derivate, insbesondere das Produkt VITAMIN E TPGS (d-Alpha Tocoperyl Polyethylene Glycol 1000 Succinate, Eastman).
- Polyethoxylierte Hydroxyfettsäureester, insbesondere das Produkt SOLUTOL HS 15 (Polyoxyethylen-660-Hydroxystearat, BASF).
- Umesterungsprodukte von natürlichen Pflanzenölglyceriden und Polyethylenpolyolen. Hierzu gehören Umesterungsprodukte aus verschiedenen, beispielsweise nichthydrierten, Pflanzenölen, wie Maisöl, Kernöl, Mandelöl, Erdnussöl, Olivenöl und Palmenöl, sowie aus Gemischen hiervon mit Polyethylenglykolen, insbesondere mit solchen, die ein mittleres Molekulargewicht von 200-800 haben. Verschiedene derartige Umesterungsprodukte sind unter der Bezeichnung LABRAFIL bekannt und erhältlich (Fiedler, Lexikon der Hilfsstoffe, 3. Auflage, Seite 707, 1989); hiervon sind die Produkte LABRAFIL M 1944 CS und LABRAFIL M 2130 CS besonders geeignet.
- Ethylenoxidaddukte von Sterolen und Derivaten davon, beispielweise von Cholesterol und Derivaten hiervon, wie Produkte, die von Sitosterol, Campesterol, oder Stigmasterol abgeleitet sind, beispielsweise von Sojasterolen und Derivaten hiervon (Fiedler, Lexikon der Hilfsstoffe, 3. Auflage, Seiten 554 und 555, 1989), wie sie unter den Bezeichnungen GENEROL bekannt und erhältlich sind, insbesondere die Produkte GENEROL 122 E5, 122 E10 und 122 E25.

Die erfindungsgemässen Mikroemulsion-Prekonzentrate umfassen sowohl Systeme, die ein einzelnes oberflächenaktives Mittel enthalten, als auch Systeme, die ein Gemisch von zwei oder mehreren oberflächenaktiven Mitteln enthalten, z.B. TWEEN 80 + CREMOPHOR RH 40, TWEEN 80 + CREMOPHOR RH 40 + VITAMIN E TPGS etc.

Erfindungsgemäss verwendet man vorzugsweise eine oberflächenaktive Komponente, welche einen Polyoxyethylensorbitanfettsäureester, ein polyoxyethylen-glykoliertes natürliches oder hydriertes Pflanzenöl oder Mischungen davon enthält.

Die erfindungsgemässen Mikroemulsion-Prekonzentrate können auch noch weitere Stoffe enthalten, wie z.B. Antioxidantien, Verdikkungsmittel, Riech- und/oder Geschmacksstoffe, Farbstoffe, etc.

Als Antioxidantien eignen sich beispielsweise Ascorbylpalmitat, Butylhydroxyanisol (BHA), Butylhydroxytoluol (BHT) und Tocopherole, z.B. D,L-alpha-Tocopherol (Vitamin E).

Als verdickungsmittel eignen sich verschiedene pharmazeutisch annehmbare polymere und anorganische Materialien, wie
- Polyacrylatharze und copolymere Polyacrylatharze, wie sie beispielsweise unter den Bezeichnungen CARBOPOL und EUDRAGIT bekannt und erhältlich sind (Fiedler, Lexikon der Hilfsstoffe, 3. Auflage, Seiten 254 bis 256 bzw. 486 bis 487, 1989), insbesondere die Produkte CARBOPOL 934, 940 und 941 bzw. EUDRAGIT E, L, S, RL und RS.
- Cellulosen und Cellulosederivate, wie Methylcellulose, Ethylcellulose, Propylcellulose, Hydroxypropylcellulose, Hydroxypropylmethylcellulose, Celluloseacetat, Celluloseacetatphthalat, Celluloseacetatsuccinat und Hydroxypropylmethylcellulosephthalat sowie Salze hiervon. Beispiele für solche Produkte sind die unter den Bezeichnungen KLUCEL und METHOCEL bekannten und erhältlichen Produkte (Fiedler, Lexikon der Hilfsstoffe, 3. Auflage, Seiten 688 und 790, 1989), insbesondere die Produkte KLUCEL LF, MF, GF und HF und METHOCEL K 100, K 15M, K 100M, E 5M, E 15, E 15M und E 100M.
- Polyvinylpyrrolidone, einschliesslich beispielsweise Poly-N-vinylpyrrolidone und Vinylpyrrolidoncopolymerisate, wie Vinylpyrrolidon-Vinylacetat-Copolymerisate. Beispiele für solche Produkte sind unter der Bezeichnung KOLLIDON (in den USA auch als POVIDONE bezeichnet) bekannt und erhältlich (Fiedler, Lexikon der Hilfsstoffe, 3. Auflage, Seiten 694 bis 696, 1989), insbesondere die Produkte KOLLIDON 30 und 90.
- Polyvinylharze, einschliesslich beispielsweise Polyvinylacetate und Polyvinylalkohole, sowie auch andere polymere Materialien, wie Tragacanthgummi, Gummi arabicum, und Alginate, z.B. Alginsäure und Salze davon, wie Natriumalginat.
- Anorganische Verdickungsmittel, wie Attapulgit, Bentonit und Silikate, einschliesslich hydrophobe Siliciumdioxide, wie alkylierte, beispielsweise methylierte Siliciumdioxidgele, insbesondere kolloidale Siliciumdioxide, wie sie unter der Bezeichnung AEROSIL bekannt und erhältlich sind (Handbook of Pharmaceutical Excipients, Herausgeber American Pharmaceutical Association/The Pharmaceutical Society of Great Britain, Seiten 253 bis 256, 1986), insbesondere die Produkte AEROSIL 130, 200, 300, 380, 0, OX 50, TT 600, MOX 80, MOX 170, LK 84 und das methylierte AEROSIL R 972.

Falls geplant ist, insbesondere zwecks oraler Verabreichung, dass die erfindungsgemässen Zusammensetzungen für eine direkte Verabreichung geeignete Enddosierungsformen darstellen sollen, dann lassen sich durch die vorliegende Erfindung auch pharmazeutische Zusammensetzungen bereitstellen, die einen in Wasser schwerlöslichen, in Komponente (a) und/oder (b) jedoch löslichen therapeutischen Wirkstoff enthalten und die selbst Mikroemulsionen darstellen; in diesen Mikroemulsionen soll der Wirkstoff stabil solubilisiert sein, wobei über mehrere Wochen keine Präzipitate beobachtet werden. Für eine orale Verbreichung können Mikroemulsionen, die man beispielsweise durch Verdünnen der erfindungsgemässen Mikroemulsion-Prekonzentrate mit Wasser oder einem wässrigen Medium erhält, direkt als Trinkformulierungen verwendet werden. Ist eine topische oder parenterale Anwendung vorgesehen, dann enthalten Zusammensetzungen, in denen weitere Hilfsstoffe vorhanden sein können, ebenfalls Wasser, so dass sich eine wässrige Mikroemulsion in Form eines Sprays, Gels, einer Paste, einer Creme, einer Injektionslösung, einer Infusionslösung oder dergleichen ergibt. Solche pharmazeutischen Zusammensetzungen in Form von Mikroemulsionen sind ebenfalls neu und Gegenstand der vorliegenden Erfindung.

Die erfindungsgemässen Mikroemulsionen sind vor allem dadurch gekennzeichnet, dass sie erhältlich sind durch Vermischen eines Mikroemulsion-Prekonzentrats enthaltend
(a) eine Mischung bestehend aus einem mittelkettigen Triglycerid und einer Omega-9-Fettsäure und/oder einer Omega-6-Fettsäure;
(b) eine oberflächenaktive Komponente enthaltend ein Tensid vom Polyoxethylen-Typ
(c) einen in Wasser schwerlöslichen, in Komponente (a) und/oder (b) jedoch löslichen wirkstoff, beispielsweise ein Cyclosporin;
(d) mit Wasser oder einem wässrigen Medium.

Die Zusammensetzung soll im wesentlichen frei sein von mit Wasser mischbaren oder in Wasser löslichen Komponenten; dabei handelt es sich vor allem um:
(1) C₁-C₅-Alkyl- oder Tetrahydrofurfuryl-Diether oder -Teilether niedermolekularer Mono- oder Polyoxy-C₂-C₁₂-Alkandiole;
(2) 1,2-Propylenglykol;
(3) niedere Alkanole;
(4) veresterungsprodukte von Polycarbonsäuren mit 2-10, insbesondere 3-5 Carboxylgruppen mit C₁-C₁₀-Alkoholen; und
(5) Veresterungsprodukte von Polyolen mit 2-10, insbesondere 3-5 Carboxylgruppen mit C₂-C₁₁-Carbonsäuren;

Je nach der Menge des vorhandenen Wassers handelt es sich um W/O-Mikroemulsionen, um bikontinuierliche Mikroemulsionen oder O/W-Mikroemulsionen.

Die erfindungsgemässsen Mikroemulsionen vom O/W-Typ (Öl-in-Wasser) weisen Stabilitätseigenschaften auf, wie sie weiter oben im Zusammenhang mit Mikroemulsionen beschrieben worden sind, d.h. insbesondere, dass in diesen Mikroemulsionen der Wirkstoff stabil solubilisiert ist und über mehrere Wochen kein Präzipitat beobachtet werden kann. Die Teilchengrösse dieser Mikroemulsionen ist kleiner als 150 nm, vorzugsweise kleiner als 100 nm.

Die erfindungsgemässen Mikroemulsion-Prekonzentrate und Mikroemulsionen eignen sich als Vehikel für in Wasser schwerlösliche, in Komponente (a) und/oder (b) jedoch lösliche Stoffe, namentlich pharmazeutische, biotechnologische und kosmetische Wirkstoffe sowie Lebensmittel-Wirkstoffe und Zell- oder Gewebekultur-Wirkstoffe. Die erfindungsgemässen Mikroemulsion-Prekonzentrate können Wirkstoffe auch in fein dispergierter Form enthalten. In ihrer Wasserphase können die erfindungsgemässen Mikroemulsionen wasserlösliche Substanzen enthalten, z.B. wasserlösliche Wirkstoffe.

Ein Beispiel eines in Wasser schwerlöslichen, in Komponente (a) und/oder (b) jedoch löslichen pharmazeutischen Wirkstoffs ist das Kapillartherapeutikum Quercetin. Die erfindungsgemässen Mikroemulsion-Prekonzentrate und Mikroemulsionen können aber auch als Vehikel für andere in Wasser schwerlösliche, in Komponente (a) und/oder (b) jedoch lösliche pharmazeutische Wirkstoffe dienen.

Ein Beispiel eines in Wasser schwerlöslichen, in Komponente (a) und/oder (b) jedoch löslich kosmetischen Wirkstoffes ist das Antifaltenmittel Coenzym Q10. Flavonoide, UV-Filter und Vitamine sind weitere Beispiele kosmetischer Wirkstoffe.

Beispiele biotechnologischer Wirkstoffe sind Peptide und Proteine, insbesondere rekombinante Proteine.

Lebensmittel-Wirkstoffe sind Nährstoffe, die auch einen präventiven oder gesundheitlichen Nutzen aufweisen. Beispiele solcher Lebensmittel-Wirkstoffe sind Omega-3-Fettsäuren (wie EPA und DHA) und Vitamine.

Beispiele von Zell- oder Gewebekultur-Wirkstoffen sind Vitamine und Retinoide.

Mikroemulsion-Prekonzentrate und Mikroemulsionen, welche in Wasser schwerlösliche, in Komponente (a) und/oder (b) jedoch lösliche wirkstoffe enthalten, können zur Verabreichung in irgendeiner geeigneten Weise angewandt werden, beispielsweise oral in einem Formkörper oder einer Einheitsdosierungsform, insbesondere einer Weich- oder Hartgelatinekapsel; Für diese Verabreichung erweisen sich die erfindungsgemässen Zusammensetzungen als besonders geeignet, weil eine Zugabe von flüchtigen organischen Lösungsmitteln, insbesondere vom häufig verwendeten Ethanol nicht erforderlich ist. Beim Einsatz von flüchtigen organischen Lösungsmitteln wird durch das Verdampfen des Lösungsmittels durch die Aussenwand des Formkörpers, insbesondere der weich- oder Hartgelatinekapsel, die Lagerfähigkeit negativ beeinträchtigt und der Wirkstoff kristallisiert aus. Das Eintreten dieser negativen Effekte muss durch aufwendige Massnahmen bei der Verpackung und Lagerung vermieden werden.

Weitere geeignete Formen der Verabreichung sind beispielsweise irparenteral oder topisch, beispielsweise zur Anwendung auf der Haut, wie als Spray, Creme, Paste, Lotion, Gel, Salbe, Breiumschlag, Kataplasma, Pflaster, Hautauflage und dergleichen; oder für eine ophthalmische Anwendung, beispielsweise in Form von Augentropfen, Augenlotionen oder Augengelen. Leicht fliessfähige Formen können auch beispielsweise für eine intraläsionale Injektion verwendet oder rektal verabreicht werden, beispielsweise als ein Enema. Sie sind jedoch in erster Linie für eine orale, parenterale oder topische Anwendung gedacht, wobei im Fall einer topischen Anwendung insbesondere eine Anwendung auf der Haut in Betracht kommt.

Für orale Verabreichung bestimmte, einen in Wasser schwerlöslichen, in Komponente (a) und/oder (b) jedoch löslichen Wirkstoff enthaltende Mikroemulsion-Prekonzentrate können bei Kontakt mit Wasser eine Mikroemulsion mit einer durchschnittlichen Teilchengrösse von unter 150 nm, vorzugsweise unter 100 nm bilden. Mikroemulsionen, die man beispielsweise durch Verdünnen der Mikroemulsion-Prekonzentrate mit Wasser oder einem wässrigen Medium erhält, können direkt als Trinkformulierungen verwendet werden. Ist eine topische oder parenterale Anwendung vorgesehen, dann enthalten Zusammensetzungen, in denen weitere Hilfsstoffe vorhanden sein können, ebenfalls Wasser, so dass sich eine wässrige Mikroemulsion in Form eines Sprays, Gels, einer Paste, einer Creme, einer Injektionslösung, einer Infusionslösung oder dergleichen ergibt.

Bevorzugte, für topische, insbesondere dermale Anwendung bestimmte Ausführungsformen der erfindungsgemässen Mikroemulsion-Prekonzentrate und Mikroemulsionen enthalten zusätzlich eine oder mehrere der folgenden Komponenten:
- Feste Kohlenwasserstoffe, beispielsweise Erdölgele, wie Weisspetrolatum oder VASELINE, Ceresin und feste Paraffine, und auch Wachse, einschliesslich tierischer, pflanzlicher und synthetischer Wachse, wie Spermacetwachs, Carnaubawachs und Bienenwachs.
- Flüssige Kohlenwasserstoffe, beispielsweise flüssige Paraffine, und Fettsäureester, wie Isopropylmyristat und Cetylpalmitat.
- Nichtflüchtige Silicone, einschliesslich Siliconöle und Siliconpasten, sowie Copolymerisate aus Silicon und Polyalkylenoxid (Fiedler, Lexikon der Hilfsstoffe, 3. Auflage, Seiten 1109 und 1110, 1989), insbesondere die unter der Bezeichnung PIROETHICON bekannten und erhältlichen Produkte.

Durch Anwendung solcher Zusätze oder von Gemischen davon lassen sich Emulsionen in flüssiger oder halbfester Form erhalten, was beispielsweise von den für eine topische Anwendung gewünschten Bedürfnissen abhängt.

Zweckmässigerweise enthalten die für topische, insbesondere dermale Anwendung bestimmten Zusammensetzungen zusätzlich
- ein Antioxidationsmittel, wie Ascorbylpalmitat, Butylhydroxyanisol (BHA), Butylhydroxytoluol (BHT) und Tocopherole, z.B. D,L-alpha-Tocopherol (Vitamin E);
- ein antibakterielles Mittel, wie Benzylalkohol, Methylparaben, Propylparaben, Benzalkoniumchlorid, Benzoesäure, Sorbinsäure oder Chlorbutanol;
- einen Stabilisator, wie mikrokristalline Stärke, Natrium-EDTA, oder Magnesiumsulfat;
- ein Mittel zur Verbesserung der Hautpenetration, beispielsweise 1-Dodecylazacycloheptan-2-on, das auch als AZON bekannt ist (Fiedler, Lexikon der Hilfsstoffe, 3. Auflage, Seite 190, 1989).

Eine besonders bevorzugte, für dermale Verabreichung bestimmte Ausführungsform einer erfindungsgemässen Zusammensetzung ist dadurch gekennzeichnet, dass sie erhältlich ist durch Verwendung einer Komponente (b) enthaltend einen Polyoxyethylensorbitanfettsäureester und/oder ein polyoxyethylenglykoliertes natürliches oder hydriertes Pflanzenöl.

Die erfindungsgemässen Mikroemulsion-Prekonzentrate können hergestellt werden, indem man die einzelnen Bestandteile, gegebenenfalls unter Erwärmen, innig miteinander vermischt. Die Mikroemulsion-Prekonzentrate können auch hergestellt werden, indem man die Komponente (b) unter Rühren, gegebenenfalls unter Erwärmen, in der Komponente (a) löst, und die erhaltene Lösung unter weiterem Rühren mit der Komponente (c) versetzt.

Die erfindungsgemässen Mikroemulsionen lassen sich aus den Mikroemulsion-Prekonzentraten durch Verdünnen mit Wasser oder anderen wässrigen Flüssigkeiten herstellen.

Erfindungsgemässe Mikroemulsion-Prekonzentrate, welche für orale Verabreichung geeignet sind, liegen zweckmässigerweise in Einheitsdosierungsform vor, z.B. in einer weich- oder Hartgelatinekapsel, und sie enthalten zweckmässigerweise 0.5 bis 20, vorzugsweise 5-15 Gewichtsprozent eines in Wasser schwerlöslichen, in Komponente (a) und/oder (b) jedoch löslichen Wirkstoffs, beispielsweise ein Cyclosporin (Komponente (c)), 9,5 bis 70, vorzugsweise 20 bis 70 Gewichtsprozent und weiter breiter bevorzugt 25 bis 65 Gewichtsprozent einer Mischung bestehend aus einem mittelkettigen Triglycerid und einer Omega-9-Fettsäure und/oder einer Ömega-6-Fettsäure (Komponente (a)) und 20 bis 90, vorzugsweise 25 bis 65 Gewichtsprozent der oberflächenaktiven Komponente (b).

Erfindungsgemässe Mikroemulsion-Prekonzentrate, welche für topische oder parenterale Anwendung geeignet sind, enthalten zweckmässigerweise 0.01 bis 15 Gewichtsprozent eines in Wasser schwerlöslichen, in Komponente (a) und/oder (b) jedoch löslichen Wirkstoffs, beispielsweise ein Cyclosporin.

Vorzugsweise ist das Mengenverhältnis von Omega-9-Fettsäure und/oder Omega-6-Fettsäure zum mittelkettigen Triglycerid 1:1 bis 1:200, besonders bevorzugt 1:2 bis 1:20.

In den erfindungsgemässen Cyclosporin Zusammensetzungen beträgt das Mengenverhältnis von Omega-9-Fettsäure und/oder Omega-6-Fettsäure zum Wirkstoff insbesondere 0.5:1, vorzugsweise minde-stens 1:1. In anderen Fällen von in Wasser schwerlöslichen, in Komponente (a) und/oder (b) jedoch löslichen Wirkstoffen kann das obige Mengenverhältnis kleiner 0.5:1 sein.

Durch die folgenden Beispiele werden die erfindungsgemässen Zusammensetzungen weiter erläutert. Die Beispiele 1.1 bis 1.22 zeigen die Herstellung von Cyclosporin Zusammensetzungen in oralen Einheitsdosierungsformen, die sich beispielsweise zur Verhinderung einer Abstossung von Transplantaten oder zur Behandlung von Autoimmunkrankheiten eignen. Beispiele 2.1 bis 2.4 zeigen die Herstellung von Cyclosporin Zusammensetzungen für eine topische Anwendung, die sich beispielsweise zur Behandlung von atopischer Dermatitis oder Kontaktdermatitis, Psoriasis, Haarausfall oder bestimmter Augenleiden wie Konjunktivitis, Uveitis oder Keratitis eignen. Beispiel 3.1 zeigt die Herstellung einer Cyclosporin Zusammensetzung für eine parenterale Anwendung, die sich beispielsweise zur Verhinderung einer Abstossung von Transplantaten eignet.

Die Beispiele werden unter besonderer Bezugnahme auf Cyclosporin A beschrieben. Durch Anwendung anderer geeigneter wirkstoffe, insbesondere auch anderer Cyclosporine, lassen sich jedoch vergleichbare Zusammensetzungen herstellen.

Das Beispiel 4 zeigt die Herstellung eines Cyclosporin-Placebo Mikroemulsion-Prekonzentrats zur oralen Applikation und einer Cyclosporin-Placebo O/W-Mikroemulsion zur topischen oder parenteralen Applikation.

Das Beispiel 5 zeigt die Herstellung eines Quercetin Mikroemulsion-Prekonzentrats zur oralen Applikation sowie die Herstellung einer Quercetin O/W-Mikroemulsion zur topischen oder parenteralen Applikation.

### Beispiel 1: Herstellung oraler Cyclosporin A Dosierungsformen vom Typ Mikroemulsion-Prekonzentrat

### Beispiel 1.1

| | |
|---|---|
| Cyclosporin A (c) | 10.00% |
| Miglyol 812 (a1) | 35.00% |
| Oelsäure (a2) | 10.00% |
| Tween 80 (b1) | 33.75% |
| Cremophor RH 40 (b2) | 11.25% |

Das Cyclosporin A wird unter Rühren bei Raumtemperatur in den Komponenten a1 und a2 gelöst, und die erhaltene Lösung wird unter weiterem Rühren mit den Komponenten b1 und b2 versetzt. Das gebildete Mikroemulsion-Prekonzentrat wird in eine Weich- oder Hartgelatinekapsel abgefüllt. Hartgelatinekapseln werden vorzugsweise unter Benutzung des Quasi-Seal-Verfahren abgedichtet. Alternativ kann das Mikroemulsion-Prekonzentrat auch in einen Dispenser abgefüllt werden. In diesem Fall stellt der Patient durch entsprechende verdünnung mittels Wasser oder einer anderen wässrigen Flüssigkeit aus dem Mikroemulsion-Prekonzentrat eine orale Trinklösung vom Typ O/W-Mikroemulsion her.

In analoger Weise lassen sich auch folgende Zusammensetzungen herstellen und in Kapseln oder in Dispenser abfüllen.

### Beispiel 1.2

| | |
|---|---|
| Cyclosporin A (c) | 10.00% |
| Miglyol 812 (a1) | 30.00% |
| Oelsäure (a2) | 15.00% |
| Tween 80 (b1) | 33.75% |
| Cremophor RH 40 (b2) | 11.25% |

### Beispiel 1.3

| | |
|---|---|
| Cyclosporin A (c) | 10.00% |
| Miglyol 812 (a1) | 27.50% |
| Oelsäure (a2) | 17.50% |
| Tween 80 (b1) | 33.75% |
| Cremophor RH 40 (b2) | 11.25% |

### Beispiel 1.4

| | |
|---|---|
| Cyclosporin A (c) | 10.00% |
| Miglyol 812 (a1) | 25.00% |
| Oelsäure (a2) | 20.00% |
| Tween 80 (b1) | 33.75% |
| Cremophor RH 40 (b2) | 11.25% |

### Beispiel 1.5

| | |
|---|---|
| Cyclosporin A (c) | 12.50% |
| Miglyol 812 (a1) | 32.50% |
| Oelsäure (a2) | 10.00% |
| Tween 80 (b1) | 33.75% |
| Cremophor RH 40 (b2) | 11.25% |

### Beispiel 1.6

| | |
|---|---|
| Cyclosporin A (c) | 12.50% |
| Miglyol 812 (a1) | 27.50% |
| Oelsäure (a2) | 15.00% |
| Tween 80 (b1) | 33.75% |
| Cremophor RH 40 (b2) | 11.25% |

### Beispiel 1.7

| | |
|---|---|
| Cyclosporin A (c) | 15.00% |
| Miglyol 812 (a1) | 25.00% |
| Oelsäure (a2) | 15.00% |
| Tween 80 (b1) | 33.75% |
| Cremophor RH 40 (b2) | 11.25% |

### Beispiel 1.8

| | |
|---|---|
| Cyclosporin A (c) | 10.00% |
| Miglyol 818 (a1) | 30.00% |
| Oelsäure (a2) | 15.00% |
| Tween 80 (b1) | 33.75% |
| Cremophor RH 40 (b2) | 11.25% |

### Beispiel 1.9

| | |
|---|---|
| Cyclosporin A (c) | 10.00% |
| Miglyol 812 (a1) | 35.00% |
| Linolsäure (a2) | 10.00% |
| Tween 80 (b1) | 33.75% |
| Cremophor RH 40 (b2) | 11.25% |

### Beispiel 1.10

| | |
|---|---|
| Cyclosporin A (c) | 10.00% |
| Miglyol 812 (a1) | 30.00% |
| Linolsäure (a2) | 15.00% |
| Tween 80 (b1) | 33.75% |
| Cremophor RH 40 (b2) | 11.25% |

### Beispiel 1.11

| | |
|---|---|
| Cyclosporin A (c) | 12.50% |
| Miglyol 812 (a1) | 27.50% |
| Linolsäure (a2) | 15.00% |
| Tween 80 (b1) | 33.75% |
| Cremophor RH 40 (b2) | 11.25% |

### Beispiel 1.12

| | |
|---|---|
| Cyclosporin A (c) | 15.00% |
| Miglyol 812 (a1) | 25.00% |
| Linolsäure (a2) | 15.00% |
| Tween 80 (b1) | 33.75% |
| Cremophor RH 40 (b2) | 11.25% |

### Beispiel 1.13

| | |
|---|---|
| Cyclosporin A (c) | 10.00% |
| Miglyol 812 (a1) | 30.00% |
| Oelsäure (a2) | 15.00% |
| Tween 80(b1) | 25.00% |
| Cremophor RH 40 (b2) | 10.00% |
| Vitamin E TPGS (b3) | 10.00% |

### Beispiel 1.14

| | |
|---|---|
| Cyclosporin A (c) | 10.00% |
| Miglyol 810 (a1) | 25.00% |
| Oelsäure (a2) | 15.00% |
| Tween 80 (b1) | 33.75% |
| Cremophor EL (b2) | 11.25% |

### Beispiel 1.15

| | |
|---|---|
| Cyclosporin A (c) | 10.00% |
| Miglyol 812 (a1) | 25.00% |
| Linolsäure (a2) | 15.00% |
| Tween 80 (b1) | 33.75% |
| Cremophor EL (b2) | 11.25% |

### Beispiel 1.16

| | |
|---|---|
| Cyclosporin A (c) | 10.00% |
| Miglyol 812 (a1) | 30.00% |
| Oelsäure (a2) | 15.00% |
| Tween 80 (b1) | 25.00% |
| Cremophor RH 40 (b2) | 10.00% |
| Labrafil M 1944 CS (b3) | 10.00% |

### Beispiel 1.17

| | |
|---|---|
| Cyclosporin A (c) | 10.00% |
| Miglyol 812 (a1) | 30.00% |
| Oelsäure (a2) | 15.00% |
| Tween 80 (b1) | 22.50% |
| Cremophor RH 40 (b2) | 22.50% |

### Beispiel 1.18

| | |
|---|---|
| Cyclosporin A (c) | 10.00% |
| Miglyol 812 (a1) | 30.00% |
| Linolsäure (a2) | 15.00% |
| Tween 80 (b1) | 22.50% |
| Cremophor EL(b2) | 22.50% |

### Beispiel 1.19

| | |
|---|---|
| Cyclosporin A (c) | 10.00% |
| Miglyol 812 (a1) | 29.80% |
| Oelsäure (a2) | 15.00% |
| Tween 80 (b1) | 33.75% |
| Cremophor RH 40 (b2) | 11.25% |
| D,L-alpha-Tocopherol^{*)} | 0.20% |

| | |
|---|---|
| ^{*)} Antioxidationsmittel | |

### Beispiel 1.20

| | |
|---|---|
| Cyclosporin A (c) | 10.00% |
| Miglyol 812 (a1) | 29.95% |
| Oelsäure (a2) | 15.00% |
| Tween 80 (b1) | 33.75% |
| Cremophor RH 40 (b2) | 11.25% |
| Ascorbylpalmitat^{*)} | 0.05% |

| | |
|---|---|
| ^{*)} Antioxidationsmittel | |

### Beispiel 1.21

| | |
|---|---|
| Cyclosporin A (c) | 5.00% |
| Miglyol 812 (a1) | 40.00% |
| Oelsäure (a2) | 10.00% |
| Tween 80 (b1) | 33.75% |
| Cremophor RH 40 (b2) | 11.25% |

### Beispiel 1.22

| | |
|---|---|
| Cyclosporin A (c) | 2.50% |
| Miglyol 812 (a1) | 42.50% |
| Oelsäure (a2) | 10.00% |
| Tween 80 (b1) | 33.75% |
| Cremophor RH 40 (b2) | 11.25% |

Werden Zusammensetzungen obiger Art mit Wasser oder einem anderen wässrigen Medium z.B. auf 1:100 verdünnt, entstehen Mikroemulsionen, die im Falle repräsentativer Beispiele die folgenden Teilchengrössen aufweisen:

| Zusammensetzung Mikroemulsion-Prekonzentrat | O/W-Mikroemulsion | |
|---|---|---|
| | Teilchendurchmesser¹⁾ [nm] | Standardabweichung¹⁾ [nm] |
| Beispiel 1.1 | 29.0 | 14.0 |
| Beispiel 1.2 | 31.7 | 12.0 |
| Beispiel 1.3 | 32.0 | 12.3 |
| Beispiel 1.4 | 68.3 | 30.7 |
| Beispiel 1.5 | 29.5 | 13.0 |
| Beispiel 1.6 | 52.3 | 25.6 |
| Beispiel 1.7 | 81.6 | 55.4 |
| Beispiel 1.10 | 34.1 | 10.0 |
| Beispiel 1.13 | 67.7 | 31.0 |
| Beispiel 1.14 | 34.7 | 9.6 |
| Beispiel 1.19 | 32.6 | 12.1 |
| Beispiel 1.20 | 31.9 | 11.5 |

| | | |
|---|---|---|
| (1) Die Teilchendurchmesser und Teilchengrössenverteilung (Gauss) werden mittels Laserlight Scattering bestimmt (Nicomp 370 Submicron Particle Sizer, Volume weighting). | | |

Aus nachfolgender Tabelle ist ersichtlich, dass im Falle repräsentativer Beispiele die aus den Mikroemulsion-Prekonzentraten gebildeten Cycolosporin A Mikroemulsionen bezüglich Partikelgrösse und Verteilung eine ausgezeichnete Lagerstabilität aufweisen. Des weiteren können während dieser Lagerung keine Cyclosporin A Präzipitate beobachtet werden.

| Zusammensetzung Mikroemulsion-Prekonzentrat | O/W-Mikroemulsion | | | |
|---|---|---|---|---|
| | Lagerbedingungen | | Partikeldurchmesser¹⁾ [nm] | Standardabweichung¹⁾ [nm] |
| | Zeit [Monate] | Temp. [°C] | | |
| Beispiel 1.19 | 0 | | 32.6 | 12.1 |
| | 1 | 7 | 34.6 | 13.3 |
| | 1 | 25 | 35.5 | 13.4 |
| | 1 | 40 | 37.5 | 13.3 |
| Beispiel 1.20 | 0 | | 31.9 | 11.5 |
| | 1 | 7 | 33.6 | 14.0 |
| | 1 | 25 | 35.9 | 14.3 |
| | 1 | 40 | 36.4 | 13.9 |

Die Teilchendurchmesser und Teilchengrössenverteilung werden mittels Laserlight Scattering bestimmt (Nicomp 370 Submicron Particle Sizer, Volume weighting).

### Beispiel 2: Herstellung topisch anwendbarer Cyclosporin A Formen vom Typ Mikroemulsion

Die in Beispiel 1.1 bis 1.22 beschriebenen Mikroemulsion-Prekonzentrate dienen nachfolgend als Basis zur Herstellung von Sprays, Gelen, Cremen und anderen topischen Darreichungsformen, indem sie mit weiteren Additiven wie Wasser, Verdickungsmitteln und dergleichen kombiniert werden.

### Beispiel 2.1: Cyclosporin A 0.25% Mikroemulsions Pumpspray

| | |
|---|---|
| Mikroemulsion-Prekonzentrat gemäss Beisp. 1.21 | 5.00% |
| Na₂-EDTA | 0.10% |
| Benzalkoniumchlorid | 0.01% |
| 10 mM Phosphatbuffer pH 6 ad | 100.00% |

Das Mikroemulsion-Prekonzentrat wird unter Rühren bei Raumtemperatur dem Phosphatpuffer, enthaltend Na₂-EDTA und Benzalkoniumchlorid, zugesetzt. Die resultierende Cyclosporin A O/W-Mikroemulsion wird in einen Pumpspray abgefüllt. Anstelle des Pumpspray kommen auch Druckgas- oder Aerosolsprays in Frage.

### Beispiel 2.2: Cyclosporin A 0.25% Hydrogel

| | |
|---|---|
| Mikroemulsion-Prekonzentrat gemäss Beispiel 1.2 | 2.50% |
| Na₂-EDTA | 0.10% |
| Benzalkoniumchlorid | 0.01% |
| Natriumcarboxymethylcellulose 450 cP | 3.50% |
| 10 mM Phosphatbuffer pH 6 ad | 100.00% |

Das Mikroemulsion-Prekonzentrat wird unter Rühren zum Phosphatpuffer, enthaltend Na₂-EDTA und Benzalkoniumchlorid, zugesetzt. Die resultierende Cyclosporin A O/W-Mikroemulsion wird in üblicher Weise mit der Natriumcarboxymethylcellulose zum Hydrogel weiter verarbeitet und konfektioniert.

### Beispiel 2.3: Cyclosporin A 0.25% O/W-Emulsion

| | |
|---|---|
| Mikroemulsion-Prekonzentrat gemäss Beispiel 1.10 | 2.500% |
| Isopropylpalmitat | 8.000% |
| Glycerylstearat | 7.000% |
| Glycerin | 5.000% |
| Steareth-2 + PEG-8 Distearate | 4.000% |
| Paraffinum liquidum | 4.000% |
| Cera microcristallina | 4.000% |
| Steareth-21 | 3.000% |
| Dimethicon | 1.000% |
| Benzylalkohol | 0.800% |
| Phenoxyethanol | 0.800% |
| Lanolinalkohol | 0.100% |
| Natriumhydroxid | 0.005% |
| Wasser ad | 100.000% |

Das Mikroemulsion-Prekonzentrat wird unter Rühren bei Raumtemperatur zur Wasserphase gegeben. Die resultierende Cyclosporin A O/W-Mikroemulsion wird in üblicher Weise mit der Oelphase zur O/W-Emulsion weiter verarbeitet und konfektioniert.

### Beispiel 2.4: Cyclosporin A 0.1% Auqentropfen

| | |
|---|---|
| Mikroemulsion-Prekonzentrat gemäss Beispiel 1.22 | 4.00% |
| Na₂-EDTA | 0.10% |
| Benzalkoniumchlorid | 0.01% |
| Isotonischer Kochsalz-Phosphatpuffer pH 7.4 ad | 100.00% |

Das Mikroemulsion-Prekonzentrat wird unter Rühren bei Raumtemperatur dem Kochsalz-Phosphatpuffer, enthaltend Na₂-EDTA und Benzalkoniumchlorid, zugesetzt. Die resultierende Cyclosporin A O/W-Mikroemulsion wird 0.2 µm steril filtriert und in Pipettenflaschen oder andere gebräuchliche Gebinde abgefüllt.

### Beispiel 3: Herstellung parenteral anwendbarer Cyclosporin A Formen vom Typ Mikroemulsion

Die in Beispiel 1.1 bis 1.22 beschriebenen Mikroemulsion-Prekonzentrate können als Basis zur Herstellung von Injektions- oder Infusionslösungen dienen, indem sie mit weiteren Additiven, wie physiologischer Kochsalzlösung oder 5% iger Glukoselösung und dergleichen, entsprechend verdünnt werden.

### Beispiel 3.1: Cyclosporin A 0.25% Infusionslösung

| | |
|---|---|
| Mikroemulsion-Prekonzentrat gemäss Beispiel 1.19 | 2.50% |
| 5%ige Glukoselösung ad | 100.00% |

Das Mikroemulsion-Prekonzentrat wird unter Rühren bei Raumtemperatur der Glukoselösung zugesetzt. Die resultierende Cyclosporin A O/W-Mikroemulsion wird 0.2 µm steril filtriert und in gebräuchliche sterile Gebinde abgefüllt.

### Beispiel 4

### Beispiel 4.1 Herstellung einer oraler Cyclosporin A-Placebo Dosierunqsform vom Typ Mikroemulsion-Prekonzentrat

| | |
|---|---|
| Cyclosporin A (c) | 0.00% |
| Miglyol 812 (a1) | 40.00% |
| Oelsäure (a2) | 15.00% |
| Tween 80 (b1) | 33.75% |
| Cremophor RH 40 (b2) | 11.25% |

Die Komponenten (a1), (a2), (b1) und (b2) werden unter Rühren, ggf unter leichtem Erwärmen, gemischt. Das gebildete Mikroemulsion-Prekonzentrat wird in eine Weich- oder Hartgelatinekapsel abgefüllt. Hartgelatinekapseln werden vorzugsweise unter Benutzung des Quasi-Seal-Verfahren abgedichtet.

### Beispiel 4.2 Herstellung eines Cyclosporin A-Placebo O/W-Mikroemulsion Pumpsprays zur topischen Applikation

| | |
|---|---|
| Mikroemulsion-Prekonzentrat gemäss Beisp. 4.1 | 5.00% |
| Na₂-EDTA | 0.10% |
| Benzalkoniumchlorid | 0.01% |
| 10 mM Phosphatbuffer pH 6 ad | 100.00% |

Das Mikroemulsion-Prekonzentrat wird unter Rühren bei Raumtemperatur dem Phosphatpuffer, enthaltend Na₂-EDTA und Benzalkoniumchlorid, zugesetzt. Die resultierende Cyclosporin A-Placebo O/W-Mikroemulsion, mit einem Teilchendurchmesser von 26.8 ± 13.4 nm, wird in einen Pumpspray abgefüllt. Anstelle des Pumpspray kommen auch Druckgas- oder Aerosolsprays in Frage.

### Beispiel 5

### Beispiel 5.1 Herstellung einer oralen Quercetin Dosierungsform vom Typ Mikroemulsion-Prekonzentrat

| | |
|---|---|
| Quercetin (c) | 1.00% |
| Miglyol 812 (a1) | 45.00% |
| Oelsäure (a2) | 9.00% |
| Tween 80 (b1) | 45.00% |

Die Komponente (a1), (a2), (b1) werden unter Rühren (z.B. Magnetrührwerk) bei Raumtemperatur gemischt. In der resultierenden klaren Flüssigkeit wird Quercetin unter Rühren (z.B. Magnetrührwerk) bei Raumtemperatur gelöst. Das gebildete Mikroemulsion-Prekonzentrat wird in eine weich- oder Hartgelatinekapsel abgefüllt. Hartgelatinekapseln werden vorzugsweise unter Benutzung des Quasi-Seal-Verfahren abgedichtet.

### Beispiel 5.2 Herstellung eines Quercetin O/W-Mikroemulsion Pumpsprays zur topischen Applikation

| | |
|---|---|
| Mikroemulsion-Prekonzentrat gemäss Beisp. 5.1 | 10.00% |
| Na₂-EDTA | 0.10% |
| Benzalkoniumchlorid | 0.01% |
| 10 mM Phosphatbuffer pH 6 ad | 100.00% |

Das Mikroemulsion-Prekonzentrat wird unter Rühren bei Raumtemperatur dem Phosphatpuffer, enthaltend Na₂-EDTA und Benzalkoniumchlorid, zugesetzt. Die resultierende Quercetin O/W-Mikroemulsion, mit einem Teilchendurchmesser von 34.1 ± 15.5 nm, wird in einen Pumpspray abgefüllt. Anstelle des Pumpspray kommen auch Druckgas- oder Aerosolsprays in Frage.

### Beispiel 6: Mikroemulsion-Prekonzentrate

### Beispiel 6.1

| | |
|---|---|
| Miglyol 812 (a1) | 40.00% |
| Oelsäure (a2) | 15.00% |
| Tween 80 (b1) | 33.75% |
| Cremophor RH 40 (b2) | 11.25% |

Die Komponenten a1, a2, b1 und b2 werden unter Rühren (z.B. Magnetrührwerk), gegebenfalls unter leichtem Erwärmen, gemischt.

In analoger Weise lassen sich auch folgende Mikroemulsion-Prekonzentrate herstellen:

### Beispiel 6.2

| | |
|---|---|
| Miglyol 812 (a1) | 40.00% |
| Oelsäure (a2) | 15.00% |
| Tween 80 (b1) | 33.75% |
| Cremophor EL (b2) | 11.25% |

### Beispiel 6.3

| | |
|---|---|
| Miglyol 812 (a1) | 45.00% |
| Oelsäure (a2) | 10.00% |
| Tween 80 (b1) | 33.75% |
| Cremophor EL (b2) | 11.25% |

### Beispiel 6.4

| | |
|---|---|
| Miglyol 812 (a1) | 50.00% |
| Oelsäure (a2) | 5.00% |
| Tween 80 (b1) | 33.75% |
| Cremophor EL (b2) | 11.25% |

### Beispiel 6.5

| | |
|---|---|
| Miglyol 812 (a1) | 45.00% |
| Oelsäure (a2) | 10.00% |
| Cremophor EL (b) | 45.00% |

### Beispiel 6.6

| | |
|---|---|
| Miglyol 812 (a1) | 50.00% |
| Oelsäure (a2) | 5.00% |
| Tween 80 (b) | 45.00% |

Die in Beispiel 1.1 bis 1.6 beschriebenen Mikroemulsion-Prekonzentrate dienen nachfolgend als Basis zur Herstellung von O/W Mikroemulsioinen.

## Patentansprüche

1. Zusammensetzung in Form eines Mikroemulsion-Prekonzentrates, enthaltend
(a) eine Mischung bestehend aus einem Fettsäuretriglycerid, in dem die Fettsäurereste 4 bis 18 Kohlenstoffatome aufweisen, und einer Omega-9-Fettsäure und/oder einer Omega-6-Fettsäure; und
(b) eine oberflächenaktive Komponente enthaltend ein Tensid vom Polyoxyethylen-Typ,
welches im Wesentlichen frei ist von mit Wasser mischbaren oder in Wasser löslichen Komponenten.

2. Zusammensetzung in Form einer Mikroemulsion erhältlich durch Vermischen eines Mikroemulsion-Prekonzentrats, enthaltend
(a) eine Mischung bestehend aus einem Fettsäuretriglycerid, in dem die Fettsäurereste 4 bis 18 Kohlenstoffatome aufweisen, und einer Omega-9-Fettsäure und/oder einer Omega-6-Fettsäure; und
(b) eine oberflächenaktive Komponente enthaltend ein Tensid vom Polyoxyethylen-Typ;
welches im Wesentlichen frei ist von mit Wasser mischbaren oder in Wasser löslichen Komponenten,
mit Wasser oder einem wässrigen Medium.

3. Zusammensetzung nach Anspruch 1 oder 2, **dadurch gekennzeichnet, dass** die mit Wasser mischbaren oder in Wasser löslichen Komponenten ausgewählt sind aus der Gruppe bestehend aus
- C₁-C₅-Alkyl- oder Tetrahydrofurfuryl-Diethern oder -Teilether n niedermolekularer Mono- oder Polyoxy-C₂-C₁₂-Alkandiole;
- 1,2-Propylenglykol;
- niederen Alkanolen;
- Veresterungsprodukten von Polycarbonsäuren mit 2-10 Carboxylgruppen mit C₁-C₁₀-Alkoholen; und
- Veresterungsprodukten von Polyolen mit 2-10 Carboxylgruppen mit C₂₋C₁₁-Carbonsäuren.

4. Zusammensetzung nach einem der Ansprüche 1 bis 3, **dadurch gekennzeichnet, dass** die Fettsäurereste des Fettsäuretriglycerids 6-18 C-Atome aufweisen.

5. Zusammensetzung nach Anspruch 4, **dadurch gekennzeichnet, dass** das mittelkettige Triglycerid ein Capryl-/Caprinsäure-Triglycerid ist.

6. Zusammensetzung nach einem der Ansprüche 1 bis 5, **dadurch gekennzeichnet, dass** die Omega-9-Fettsäure und/oder die Omega-6-Fettsäure 12-24 C-Atome aufweist.

7. Zusammensetzung nach Anspruch 6, **dadurch gekennzeichnet, dass** die Omega-9-Fettsäure Ölsäure ist.

8. Zusammensetzung nach Anspruch 6 oder 7, **dadurch gekennzeichnet, dass** die Omega-6-Fettsäure Linolsäure ist.

9. Zusammensetzung nach einem der Ansprüche 1 bis 8, **dadurch gekennzeichnet, dass** sie als Komponente (a) eine Mischung aus einem Capryl-/Caprinsäure-Triglycerid, Ölsäure und/oder Linolsäure enthält.

10. Zusammensetzung nach einem der Ansprüche 1 bis 9, **dadurch gekennzeichnet, dass** das Mengenverhältnis von Omega-9-Fettsäure und/oder Omega-6-Fettsäure zum mittelkettigen Triglycerid 1:1 bis 1:200 ist.

11. Zusammensetzung nach einem der Ansprüche 1 bis 10, **dadurch gekennzeichnet, dass** die oberflächenaktive Komponente (b) einen Polyoxyethylensorbitanfettsäureester, ein polyoxyethylen-glykoliertes natürliches oder hydriertes Pflanzenöl oder Mischungen davon enthält.

12. Zusammensetzung nach einem der Ansprüche 1 und 3 bis 11, **dadurch gekennzeichnet, dass** die Komponente (a) in einer Menge von 20 bis 70 Gewichtsprozent bezogen auf das Gesamtgewicht der Zusammensetzung vorhanden ist.

13. Zusammensetzung nach einem der Ansprüche 1 und 3 bis 12, **dadurch gekennzeichnet, dass** die oberflächenaktive Komponente (b) in einer Menge von 20 bis 90 Gewichtsprozent bezogen auf das Gesamtgewicht der Zusammensetzung vorhanden ist.

14. Zusammensetzung nach einem der Ansprüche 2 bis 11, **dadurch gekennzeichnet, dass** sie eine O/W-Mikroemulsion mit einer durchschnittlichen Teilchengrösse unter 150 nm ist.

15. Zusammensetzung nach einem der Ansprüche 1 bis 14, enthaltend als Komponente (c) einen in Komponente (a) und/oder Komponente (b) löslichen Wirkstoff.

16. Zusammensetzung nach Anspruch 15, **dadurch gekennzeichnet, dass** die Komponente (c) ausgewählt ist aus der Gruppe bestehend aus einem pharmazeutischen Wirkstoff, einem biotechnologischen Wirkstoff, einem kosmetischen Wirkstoff, einem Lebensmittelwirkstoff oder einem Zell- oder Gewebekulturwirkstoff.

17. Zusammensetzung nach Anspruch 15 oder 16, **dadurch gekennzeichnet, dass** der Wirkstoff ausgewählt ist aus der Gruppe der in Wasser nicht oder schwerlöslichen, jedoch in Komponente (a) und/oder Komponente (b) löslichen Wirkstoffe.

18. Zusammensetzung nach einem der Ansprüche 1 bis 17, enthaltend einen in Wasser löslichen Wirkstoff.

19. Zusammensetzung nach Anspruch 18, **dadurch gekennzeichnet, dass** es sich bei der Komponente c) um Coenzym Q10 handelt.

20. Verwendung einer Zusammensetzung gemäss einem der Ansprüche 15 bis 19 zur Herstellung einer pharmazeutischen Zusammensetzung unter Einsatz wenigstens eines pharmazeutischen Wirkstoffs.

21. Verwendung einer Zusammensetzung nach einem der Ansprüche 15 bis 19 zur Herstellung einer kosmetischen Zusammensetzung unter Einsatz wenigstens eines kosmetischen Wirkstoffs.

22. Verwendung einer Zusammensetzung nach einem der Ansprüche 15 bis 19 zur Herstellung einer Lebensmittel-Zusammensetzung unter Einsatz wenigstens eines Lebensmittelwirkstoffs.

23. Verwendung einer Zusammensetzung nach einem der Ansprüche 15 bis 18 zur Herstellung einer Zusammensetzung, enthaltend wenigstens ein Peptid oder Protein, insbesondere ein rekombinantes Protein.

24. Verwendung einer Zusammensetzung nach einem der Ansprüche 15 bis 18 zur Herstellung einer Zusammensetzung, enthaltend wenigstens einen Zell- oder Gewebekultur-Wirkstoff.

25. Formkörper zur Darreichung eines Wirkstoffs, enthaltend eine Zusammensetzung nach einem der Ansprüche 1 bis 19.

26. Formkörper nach Anspruch 25, **dadurch gekennzeichnet, dass** er ein Biopolymer, insbesondere Gelatine, enthält.

27. Formkörper nach einem der Ansprüche 25 oder 26, **dadurch gekennzeichnet, dass** er als Komponente c) Coenzym Q10 enthält.

28. Füllmaterial für einen Formkörper, enthaltend eine Zusammensetzung nach einem der Ansprüche 1 bis 19.

29. Trinkformulierung zur Darreichung eines Wirkstoffs enthaltend eine Zusammensetzung nach einem der Ansprüche 1 bis 19.

30. Lösung zur parenteralen Darreichung eines Wirkstoffs enthaltend eine Zusammensetzung nach einem der Ansprüche 1 bis 19.

31. Topische Darreichungsformen eines Wirkstoffs enthaltend eine Zusammensetzung nach einem der Ansprüche 1 bis 19.

32. Verwendung einer Zusammensetzung enthaltend
(a) eine Mischung bestehend aus einem Fettsäuretriglycerid, in dem die Fettsäurereste 4 bis 18 Kohlenstoffatome aufweisen, und einer Omega-9-Fettsäure und/oder einer Omega-6-Fettsäure
(b) eine oberflächenaktive Komponente enthaltend ein Tensid vom Polyöxyethylen-Typ
(c) einen Wirkstoff
als Mikroemulsion-Prekonzentrat, wobei die Ölphase und/oder das Tensid (b) aus der Gruppe der Ölphasen bzw. der Tenside so ausgewählt sind, dass der Wirkstoff (c) in der Ölphase (a) und/oder im Tensid löslich ist,
wobei das Mikroemulsion-Prekonzentrat im Wesentlichen frei ist von mit Wasser mischbaren oder in Wasser löslichen Komponenten.

33. Verwendung nach Anspruch 32, **dadurch gekennzeichnet, dass** die mit Wasser mischbaren oder in Wasser löslichen Komponenten ausgewählt sind aus der Gruppe bestehend aus
- C₁-C₅-Alkyl- oder Tetrahydrofurfuryl-Diethern oder -Teilether n niedermolekularer Mono- oder Polyoxy-C₂-C₁₂-Alkandiole;
- 1,2-Propylenglykol;
- niederen Alkanolen;
- Veresterungsprodükten von Polycarbonsäuren mit 2-10 Carboxylgruppen mit C₁-C₁₀-Alkoholen; und
- Veresterungsprodukten von Polyolen mit 2-10 Carboxylgruppen mit C₂₋C₁₁-Carbonsäuren.

34. Mikroemulsion enthaltend eine wie in Anspruch 32 definierte Zusammensetzung.

35. Formkörper enthaltend eine wie in Anspruch 32 definierte Zusammensetzung oder eine Mikroemulsion gemäss Anspruch 33.

## Claims

1. A composition in the form of a microemulsion preconcentrate containing
(a) a mixture comprising a fatty acid triglyceride in which the fatty acid residues have 4 to 18 carbon atoms, and an omega-9 fatty acid and/or an omega-6 fatty acid; and
(b) a surface-active component containing a tenside of polyoxyethylene type,
which is substantially free of water-miscible or water-soluble components.

2. A composition in the form of a microemulsion which can be obtained by mixing a microemulsion preconcentrate containing
(a) a mixture comprising a fatty acid triglyceride in which the fatty acid residues have 4 to 18 carbon atoms, and an omega-9 fatty acid and/or an omega-6 fatty acid; and
(b) a surface-active component containing a tenside of polyoxyethylene type,
which is substantially free of water-miscible or water-soluble components, with water or an aqueous medium.

3. A composition according to claim 1 or claim 2 **characterised in that** the water-miscible or water-soluble components are selected from the group consisting of
- C₁-C₅ alkyl or tetrahydrofurfuryl diethers or partial ethers of low-molecular mono- or polyoxy-C₂-C₁₂ alkane diols;
- 1,2-propylene glycol;
- low alkanols;
- esterification products of polycarboxylic acids with 2-10 carboxyl groups with C₁-C₁₀ alcohols; and
- esterification products of polyols with 2-10 carboxyl groups with C₂₋C₁₁ carboxylic acids.

4. A composition according to one of claims 1 to 3 **characterised in that** the fatty acid residues of the fatty acid triglyceride have 6-18 C-atoms.

5. A composition according to claim 4 **characterised in that** the medium-chain triglyceride is a caprylic/capric acid triglyceride.

6. A composition according to one of claims 1 to 5 **characterised in that** the omega-9 fatty acid and/or the omega-6 fatty acid has 12-24 C-atoms.

7. A composition according to claim 6 **characterised in that** the omega-9 fatty acid is oleic acid.

8. A composition according to claim 6 or claim 7 **characterised in that** the omega-6 fatty acid is linoleic acid.

9. A composition according to one of claims 1 to 8 **characterised in that** as component (a) it contains a mixture of a caprylic/capric acid triglyceride, oleic acid and/or linoleic acid.

10. A composition according to one of claims 1 to 9 **characterised in that** the quantitative ratio of omega-9 fatty acid and/or omega-6 fatty acid to the medium-chain triglyceride is 1:1 to 1:200.

11. A composition according to one of claims 1 to 10 **characterised in that** the surface-active component (b) contains a polyoxyethylene sorbitan fatty acid ester, a polyoxyethylene-glycolated natural or hydrated vegetable oil or mixtures thereof.

12. A composition according to one of claims 1 and 3 to 11 **characterised in that** the component (a) is present in an amount of 20 to 70 percent by weight with respect to the total weight of the composition.

13. A composition according to one of claims 1 and 3 to 12 **characterised in that** the surface-active component (b) is present in an amount of 20 to 90 percent by weight with respect to the total weight of the composition.

14. A composition according to one of claims 2 to 11 **characterised in that** it is an O/W microemulsion with an average particle size of less than 150 nm.

15. A composition according to one of claims 1 to 14 containing as component (c) an active substance soluble in component (a) and/or component (b).

16. A composition according to claim 15 **characterised in that** the component (c) is selected from the group consisting of a pharmaceutical active substance, a biotechnological active substance, a cosmetic active substance, a food active substance or a cell or tissue culture active substance.

17. A composition according to claim 15 or claim 16 **characterised in that** the active substance is selected from the group of active substances which are not or difficultly soluble in water but soluble in component (a) and/or component (b).

18. A composition according to one of claims 1 to 17 containing a water-soluble active substance.

19. A composition according to claim 18 **characterised in that** the component (c) is coenzyme Q10.

20. Use of a composition according to one of claims 15 to 19 for the production of a pharmaceutical composition using at least one pharmaceutical active substance.

21. Use of a composition according to one of claims 15 to 19 for the production of a cosmetic composition using at least one cosmetic active substance.

22. Use of a composition according to one of claims 15 to 19 for the production of a foodstuff composition using at least one foodstuff active substance.

23. Use of a composition according to one of claims 15 to 18 for the production of a composition containing at least one peptide or protein, in particular a recombinant protein.

24. Use of a composition according to one of claims 15 to 18 for the production of a composition containing at least one cell or tissue culture active substance.

25. A shaped body for the administration of an active substance containing a composition according to one of claims 1 to 19.

26. A shaped body according to claim 25 **characterised in that** it contains a biopolymer, in particular gelatine.

27. A shaped body according to one of claims 25 and 26 **characterised in that** as component (c) it contains coenzyme Q10.

28. A filling material for a shaped body containing a composition according to one of claims 1 to 19.

29. A drink formulation for the administration of an active substance containing a composition according to one of claims 1 to 19.

30. A solution for the parenteral administration of an active substance containing a composition according to one of claims 1 to 19.

31. Topical administration forms of an active substance containing a composition according to one of claims 1 to 19.

32. Use of a composition containing
(a) a mixture comprising a fatty acid triglyceride in which the fatty acid residues have 4 to 18 carbon atoms, and an omega-9 fatty acid and/or an omega-6 fatty acid;
(b) a surface-active component containing a tenside of polyoxyethylene type; and
(c) an active substance
as microemulsion preconcentrate wherein the oil phase and/or the tenside (b) are so selected from the group of the oil phases or the tensides respectively that the active substance (c) is soluble in the oil phase (a) and/or in the tenside,
wherein the microemulsion preconcentrate is substantially free of water-miscible or water-soluble components.

33. Use according to claim 32 **characterised in that** the water-miscible or water-soluble components are selected from the group consisting of
- C₁-C₅ alkyl or tetrahydrofurfuryl diethers or partial ethers of low-molecular mono- or polyoxy-C₂-C₁₂ alkane diols;
- 1,2-propylene glycol;
- low alkanols;
- esterification products of polycarboxylic acids with 2-10 carboxyl groups with C₁-C₁₀ alcohols; and
- esterification products of polyols with 2-10 carboxyl groups with C₂₋C₁₁ carboxylic acids.

34. A microemulsion containing a composition as defined in claim 32.

35. A shaped body containing a composition as defined in claim 32 or a microemulsion according to claim 33.

## Revendications

1. Composition sous la forme d'un préconcentré en microémulsion, contenant
(a) un mélange constitué d'un triglycéride d'acides gras, dans lequel les restes d'acides gras comportent 4 à 18 atomes de carbone, et d'un acide gras oméga-9 et/ou d'un acide gras oméga-6; et
(b) un composant tensioactif contenant un agent tensioactif du type polyoxyéthylène,
lequel est essentiellement exempt de composants miscibles à l'eau ou solubles dans l'eau.

2. Composition sous la forme d'une microémulsion pouvant être obtenue par mélange d'un préconcentré en microémulsion, contenant
(a) un mélange constitué d'un triglycéride d'acides gras, dans lequel les restes d'acides gras présentent 4 à 18 atomes de carbone, et d'un acide gras oméga-9 et/ou d'un acide gras oméga-6; et
(b) un composant tensioactif contenant un agent tensioactif du type polyoxyéthylène,
lequel est essentiellement exempt de composants miscibles à l'eau ou solubles dans l'eau,
avec de l'eau ou un milieu aqueux.

3. Composition selon la revendication 1 ou 2, **caractérisée en ce que** les composants miscibles à l'eau ou solubles dans l'eau sont choisis dans le groupe constitué par
- des diéthers ou éthers partiels d'alkyle en C₁ à C₅ ou de tétrahydrofurfuryle de mono- ou polyoxy-alcanediols en C₂ à C₁₂, de faible masse moléculaire ;
- le 1,2-propylèneglycol;
- des alcanols inférieurs;
- des produits d'estérification d'acides polycarboxyliques avec 2 à 10 groupes carboxyle avec des alcools en C₁ à C₁₀; et
- des produits d'estérification de polyols avec 2 à 10 groupes carboxyle avec des acides carboxyliques en C₂ à C₁₁.

4. Composition selon l'une des revendications 1 à 3, **caractérisée en ce que** les restes d'acides gras du triglycéride d'acides gras comportent 6 à 18 atomes de C.

5. Composition selon la revendication 4, **caractérisée en ce que** le triglycéride à chaîne moyenne est un triglycéride d'acide caprylique/acide caprique.

6. Composition selon l'une des revendications 1 à 5, **caractérisée en ce que** l'acide gras oméga-9 et/ou l'acide gras oméga-6 comporte(nt) 12 à 24 atomes de C.

7. Composition selon la revendication 6, **caractérisée en ce que** l'acide gras oméga-9 est l'acide oléique.

8. Composition selon la revendication 6 ou 7, **caractérisée en ce que** l'acide gras oméga-6 est l'acide linoléique.

9. Composition selon l'une des revendications 1 à 8, **caractérisée en ce qu'**elle contient comme composant (a) un mélange d'un triglycéride d'acide caprylique/acide caprique, d'acide oléique et/ou d'acide linoléique.

10. Composition selon l'une des revendications 1 à 9, **caractérisée en ce que** la proportion des acides gras oméga-9 et/ou des acides gras oméga-6 par rapport au triglycéride à chaîne moyenne est de 1:1 à 1:200.

11. Composition selon l'une des revendications 1 à 10, **caractérisée en ce que** le composant tensioactif (b) contient un ester d'acide et de sorbitan gras polyoxyéthylène, une huile végétale, naturelle ou hydrogénée, polyoxyéthylène glycolée ou des mélanges de ceux-ci.

12. Composition selon l'une des revendications 1 et 3 à 11, **caractérisée en ce que** le composant (a) est présent en une quantité de 20 à 70 pour cent en poids par rapport au poids total de la composition.

13. Composition selon l'une des revendications 1 et 3 à 12, **caractérisée en ce que** le composant tensioactif (b) est présent en une quantité de 20 à 90 pour cent en poids par rapport au poids total de la composition.

14. Composition selon l'une des revendications 2 à 11, **caractérisée en ce qu'**il s'agit d'une microémulsion huile/eau avec une dimension moyenne de particule inférieure à 150 nm.

15. Composition selon l'une des revendications 1 à 14, contenant comme composant (c) un principe actif soluble dans le composant (a) et/ou le composant (b).

16. Composition selon la revendication 15, **caractérisée en ce que** le composant (c) est choisi dans le groupe constitué par un principe actif pharmaceutique, un principe actif biotechnologique, un principe actif cosmétique, un principe actif alimentaire et un principe actif de culture cellulaire ou tissulaire.

17. Composition selon la revendication 15 ou 16, **caractérisée en ce que** le principe actif est choisi dans le groupe des principes actifs non solubles ou difficilement solubles dans l'eau mais solubles dans le composant (a) et/ou le composant (b).

18. Composition selon l'une des revendications 1 à 17, contenant un principe actif soluble dans l'eau.

19. Composition selon la revendication 18, **caractérisée en ce que** le composant(c) est le coenzyme Q10.

20. Utilisation d'une composition selon l'une des revendications 15 à 19 pour la préparation d'une composition pharmaceutique avec mise en oeuvre d'au moins un principe actif pharmaceutique.

21. Utilisation d'une composition- selon l'une des revendications 15 à 19 pour la préparation d'une composition cosmétique avec mise en oeuvre d'au moins un principe actif cosmétique.

22. Utilisation d'une composition selon l'une des revendications 15 à 19 pour la fabrication d'une composition alimentaire avec mise en oeuvre d'au moins un principe actif alimentaire.

23. Utilisation d'une composition selon l'une des revendications 15 à 18 pour la fabrication d'une composition contenant au moins un peptide ou au moins une protéine, en particulier une protéine recombinante.

24. Utilisation d'une composition selon l'une des revendications 15 à 18 pour la préparation d'une composition contenant au moins un principe actif de culture cellulaire ou tissulaire.

25. Objet façonné pour l'administration d'un principe actif, contenant une composition selon l'une des revendications 1 à 19.

26. Objet façonné selon la revendication 25, **caractérisé en ce qu'**il contient un biopolymère, en particulier de la gélatine.

27. Objet façonné selon l'une des revendications 25 et 26, **caractérisé en ce qu'**il contient le coenzyme Q10 en tant que composant (c).

28. Matière de charge pour un objet façonné, contenant une composition selon l'une des revendications 1 à 19.

29. Formulation buvable pour l'administration d'un principe actif, contenant une composition selon l'une des revendications 1 à 19.

30. Solution pour l'administration parentérale d'un principe actif, contenant une composition selon l'une des revendications 1 à 19.

31. Formes d'administration topique d'un principe actif, contenant une composition selon l'une des revendications 1 à 19.

32. Utilisation d'une composition contenant
(a) un mélange constitué d'un triglycéride d'acides gras, dans lequel les restes d'acides gras comportent 4 à 18 atomes de carbone, et d'un acide gras oméga-9 et/ou d'un acide gras oméga-6
(b) un composant tensioactif contenant un agent tensioactif de type polyoxyéthylène
(c) un principe actif,
en tant que préconcentré en microémulsion, la phase huileuse et/ou l'agent tensioactif (b) étant choisis dans le groupe des phases huileuses ou des agents tensioactifs, de telle manière à ce que le principe actif (c) soit soluble dans la phase huileuse (a) et/ou l'agent tensioactif, le préconcentré en microémulsion étant pour sa part essentiellement exempt de composants miscibles à l'eau ou solubles dans l'eau.

33. Utilisation selon la revendication 32, **caractérisée en ce que** les composants miscibles à l'eau ou solubles dans l'eau sont choisis dans le groupe constitué par
- des diéthers ou éthers partiels d'alkyle en C₁ à C₅ ou de tétrahydrofurfuryle de mono- ou polyoxy-alcanediols en C₂ à C₁₂, de faible masse moléculaire ;
- le 1,2-propylèneglycol;
- des alcanols inférieurs;
- des produits d'estérification d'acides polycarboxyliques avec 2 à 10 groupes carboxyle avec des alcools en C₁ à C₁₀; et
- des produits d'estérification de polyols avec 2 à 10 groupes carboxyle avec des acides carboxyliques en C₂ à C₁₁

34. Microémulsion contenant une composition telle que définie dans la revendication 32.

35. Objet façonné contenant une composition telle que définie dans la revendication 32 ou une microémulsion selon la revendication 33.
